# EUROPEAN PATENT APPLICATION

(11) **EP 1 350 837 A2**
(43) Date of publication of application: **08.10.2003**
(21) Application number: 03251701.3
(22) Date of filing: 19.03.2003
(51) Int. Cl.: C12M 1/22

(54) **Reversible petri dish**

(30) Priority: 26.03.2002 US 367209 P; 11.06.2002 US 166177
(71) Applicant: Becton Dickinson and Company, Franklin Lakes, New Jersey 07417-1880 (US)
(72) Inventor: Goff, Michael Craig, Raleigh, North Carolina 27613 (US); Hall, John P., Raleigh, North Carolina 27615 (US)
(74) Representative: Ruffles, Graham Keith

(57) **Abstract**

A reversible Petri dish having selectable structural features for different applications related to receiving, growing and examining biological entities is provided. The reversible dish can be flipped over to select the dish side having the desired structural features. The reversible dish can accommodate uses which require the entire surface area of the bottom of a dish on one side and which require a deeper volume over less surface area on the opposite side.

## Description

### BACKGROUND OF THE INVENTION

### 1. Field of the Invention

The invention relates to a petri dish having selectable structural features for different applications related to receiving, growing and examining biological entities, e.g., cells, tissues or micro-organisms. The dish can accommodate uses which require the entire surface area of the lower surface of the dish or which require a deeper volume over less surface area.

### 2. Description of Related Art

The use of petri dishes is well documented for culturing micro-organisms or for visual assay of biological entities. Petri dishes of various sizes and of differing diameter are used, depending upon the specific microbiological evaluations or techniques being performed. Dish diameters ranging from 30 to 150 mm and with edge heights ranging from 10 to 20 mm are conventional. The materials used for making petri dishes include glass, synthetic resins and, less frequently, corrosion-resistant metals.

Prior art petri dishes generally include a bottom container and a mating cover. The bottom container usually takes the form of a shallow truncated cylinder, while the cover likewise takes the form of a complementary truncated cylinder having a somewhat enlarged inside diameter to allow the cover member to fit over the bottom container. The working area for the biological sample or culture is generally the entire bottom inner surface of the bottom container. However, in many cases, it is difficult to obtain a large enough quantity of the biological material to carry out the required procedure or operation over such a large working area. For example, when working with primary cell cultures of epithelial origin or with small amounts of substrate as initial material in a relatively large series of tests, the conventional way of growing cultures in petri dishes or flasks encounters difficulties because for such test series relatively large amounts of cells or substance are necessary which often cannot be accommodated.

As such, prior art petri dishes have been structured to provide one or more smaller working areas within the petri dish. Typically, such prior art petri dishes include one or more wells formed on the bottom inner surface of the dish for accommodating relatively small amounts of biological materials or for growing so-called "micro-cultures".

It is not uncommon for a laboratory to administer microorganism cultures or perform other procedures to hundreds or even thousands of petri dishes in a single day, and this with only one or a few persons to accomplish the task. The different cultures or procedures being performed often require the use of different petri dishes, having different physical characteristics, e.g., different working areas or volumes, depending upon the amount of biological material present or specific tests being performed. Laboratory efficiency often dictates that hundreds and perhaps as many as a thousand petri dishes, often having different characteristics or structures, be used by a single technician in a single day. Such efficiency is compromised if different petri dishes, having different characteristics or structures, are required.

Therefore, there is a need for a petri dish having structural features that can accommodate different biological environments or sample sizes depending upon the particular use required.

### SUMMARY OF THE INVENTION

According to the present invention, a reversible petri dish which includes opposing dishes having different physical characteristics, e.g., different surface areas and/or volumes for receiving biological materials, is provided. The reversible petri dish can be flipped over to select the dish for use which has the desired characteristics or structure.

In one aspect, the invention relates to a reversible petri dish which includes a base wall having a first surface on one side and second surface on the opposite side; and an outer rim projecting in a first direction outwardly and substantially at a right angle from the first surface of the base wall to form a first dish interior and projecting in a second direction outwardly and substantially at a right angle from the second surface of the base wall to form a second dish interior which is diametrically opposed to the first dish interior; wherein the physical characteristics of the first and second dish interiors are different.

Different physical characteristics can include different materials, different surface textures, different surface coatings, or different surface area and/or volume for receiving biological material, for each of the dish interiors. Preferably, the surface area and/or volume for receiving biological material for the first and second dish interiors is different.

In one embodiment, at least a portion of the base wall is comprised of a rigid, transparent material. Preferably, the rigid, transparent material is a polymeric material or glass.

In another embodiment, at least one surface of the base wall supports at least one well having a well volume less than the respective dish volume. A circular well can be imbedded into the surface of the base wall so that the well has a bottom well surface below the surface of the base wall. Preferably, the bottom well surface is transparent and is comprised of glass.

In another aspect, the invention relates to a reversible petri dish which includes a surface supporting wall having a first surface on one side and a second surface on the opposite side; and an outer wall projecting in a first direction outwardly and substantially at a right angle from the first surface to a first open end to define a first dish interior, projecting in a second direction outwardly and substantially at a right angle from the second surface to a second open end to define a second dish interior, and encompassing the dish periphery. The first surface includes at least one well having an open end, a closed end and a peripheral wall therebetween defining a well interior for accommodating a biological specimen and having a periphery within the first dish periphery. The interior surface of the closed end of the well(s) defines a first working surface area and the interior surface of the second surface defines a second working surface area.

Preferably, the surface supporting wall is comprised of a rigid, transparent material. The rigid, transparent material is preferably selected from the group consisting of glass, a polymeric material and a combination thereof.

The well can be embedded into the first surface so that the open end of the well is located at the first surface and the closed end of the well, i.e., the first working surface area, is below the first surface.

In another embodiment, the surface supporting wall includes a composite of at least two layers. Preferably, the composite includes a first surface layer having a face side and a back side, and a second surface layer having a face side and a back side, in which the back side of the first layer is adhesively attached to the back side of the second layer. The face side of the first layer is the first surface of the surface supporting wall and the face side of the second layer is the second surface of the surface supporting wall. The two layers can be different materials, e.g., one layer can be a polymeric material and one layer can be glass.

A well can be formed by a hole passing through the first surface layer so that the closed end of the well is the back side of the second surface layer. The first surface layer is preferably comprised of a rigid polymeric material and the second surface layer is preferably comprised of glass. Thus, the closed end of the well can include a glass working surface area and the sidewalls of the well can be made of a polymeric material.

In yet another aspect, the invention relates to a reversible petri dish which includes a circular base wall having a first surface on one side and a second surface on the opposite side, and having at least one hole passing through the central portion of the base wall; an outer rim integrally secured to the circular base wall and encompassing the periphery of the circular base wall, in which the outer rim projects in a first direction outwardly and substantially at a right angle from the first surface of the circular base wall to form a first dish interior and projects in a second direction outwardly and substantially at a right angle from the second surface of the base wall to form a second dish interior, which is diametrically opposed to the first interior; and a circular transparent sheet having a face side and a back side, the back side being adhered to the second surface of the base wall and substantially covering the second surface. The hole passing through the base wall and the back side of the transparent sheet define a well so that the back side of the transparent sheet defines the bottom surface of the well and the hole passing through the base wall defines the side walls and open end of the well.

Preferably, the circular transparent sheet is comprised of glass and the back side of the circular sheet is adhered to the second side of the base wall using an adhesive.

### BRIEF DESCRIPTION OF THE DRAWINGS

FIG. 1 is an exploded perspective view, partly in cross-section, showing a reversible petri dish of the present invention.

FIG. 2 is a cross-sectional side elevation of the reversible petri dish of FIG. 1.

FIG. 3 is a cross sectional side elevation of another embodiment of a reversible petri dish according to the invention.

FIG. 4 is a view similar to Fig. 2 with a well imbedded in the surface of one of the dishes.

FIG. 5 is a view similar to Fig. 4 showing a well with tapered side walls.

FIG. 6 is a view similar to Fig. 4, in which the well is defined by a cylindrical wall extending away from the dish surface.

FIG. 7 is a cross-sectional side elevation of another embodiment of a reversible petri dish which contains a well in accordance with the invention.

FIG. 8 is a cross-sectional side elevation view of the embodiment depicted in Fig. 7 with a matching cover.

FIG. 9 is a cross-sectional side elevation view of a preferred embodiment structure for use with a cover.

FIG. 10 is a cross-sectional side elevation view of a preferred embodiment reversible petri dish including a composite base wall in accordance with the invention.

FIG. 11 is a view similar to FIG. 10, including a well in one of the dishes.

FIG. 12 is a cross-sectional side elevation view of another preferred embodiment of the invention.

### DETAILED DESCRIPTION OF THE INVENTION

The present invention provides a reversible petri dish which includes opposing dishes having different structural features, e.g. different volumes and/or surface areas for receiving biological materials. The reversible petri dish can be flipped over to select the dish having the desired structure.

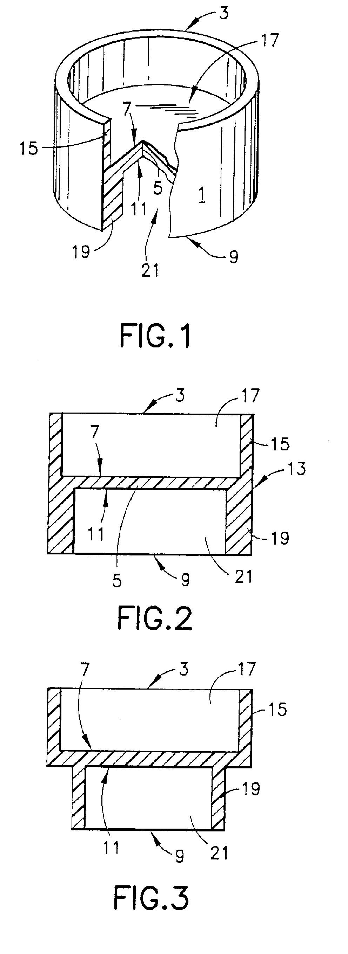

One embodiment of the invention provides a reversible petri dish that includes oppositely facing dishes sharing a common base wall, in which the dishes have different structural features. Referring to the drawings there is shown in FIGS. 1-3 a reversible petri dish **1** which includes a first dish **3** facing away from one side of the base wall **5** and having a first surface **7** for receiving biological material and a second dish **9** facing away from the opposite side of the base wall **5** and having a second surface **11** for receiving biological material, in which the surface area of the second surface **11** is less than that of the first surface **7.**

The reversible petri dish includes a circular base wall **5** and a cylindrical outer wall **13** which is integral with and encompasses the periphery of the circular base wall **5.** The cylindrical outer wall **13** includes a first section **15** which projects outwardly and substantially at a right angle from the first surface **7** of the base wall **5** to form the first dish interior **17** and includes a second section **19** which projects outwardly and substantially at a right angle from the second surface **11** of the base wall **5** to form the second dish interior **21.** The wall thickness of the second section **19** of the outer wall **13** can be greater than that of the first section **15,** resulting in less surface area for the second surface **11** within the second dish interior **21** compared to the surface area for the first surface **7** within the first dish interior **17**.

In another embodiment, the wall thickness of the first **15** and second **19** sections of the outer wall **13** can be the same or similar, but the second section of the cylindrical outer wall can have a smaller diameter (across the cylinder) than the first section of the cylindrical outer wall, as shown in Figure 3. This will again result in less surface area for the second surface **11** within the second dish interior **21** compared to the surface area for the first surface **7** within the first dish interior **17**.

The materials used for making the reversible petri dish include glass, synthetic resins (i.e., polymeric or plastic materials) and, less frequently, corrosion resistant metals. Combinations of these materials can also be used. The reversible petri dish is preferably made from glass and/or a rigid polymeric material. Typical polymeric materials include organic, preferably transparent, polymers which can be molded, using conventional molding techniques, such as polyethylene, polypropylene, polystyrene, methacrylates, polyparamethyl styrene and the like. Materials that can withstand sterilization by heating in steam without distortion or loss of transparency are preferred. Coated plastic materials, which have coatings to improve the surface characteristics of the materials, such as inorganic coatings, are also contemplated.

The base wall preferably includes a rigid, transparent material which extends at least over the surface area for receiving the biological material to facilitate the viewing of the contents of the biological sample. The rigid, transparent material is preferably glass or a polymeric material. A transparent window through the base wall preferably allows the use of both upright and inverted light microscopes to observe biological specimens or cultures contained in the dish being used. The outer wall of the dish, which faces in the opposite direction (i.e., faces downward) from the dish being used, acts as a skirt, preventing contact between the underside surface of the base wall and the underlying surface supporting the reversible petri dish. This preserves the optical quality of the petri dish base wall by reducing the possibility of scratching or soiling the underside surface of the base wall.

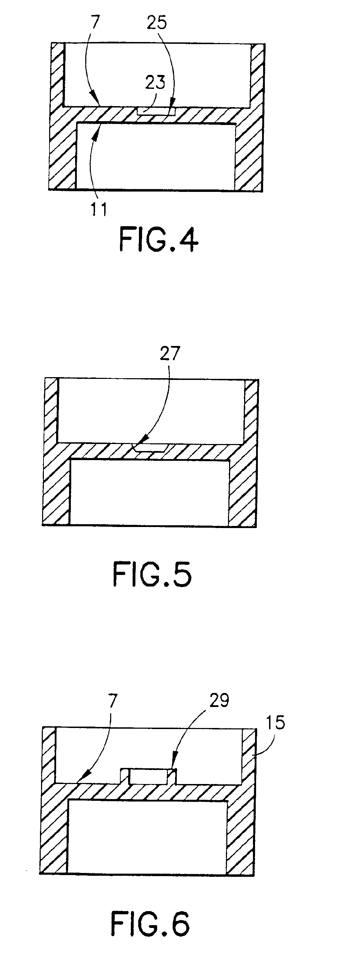

The reversible petri dish can also include at least one well for receiving smaller amounts or a deeper volume over less surface area of biological material. The well(s) can be supported by either surface of the base wall, with the well opening(s) facing in the same direction as the respective dish opening and with the well volume being less than the respective dish volume. Referring to Figure 4, a cross-sectional view of a reversible petri dish, which contains a well, is shown. The well **23** is formed by a cylindrical depression embedded into the first surface **7** of the base wall so that the well has a bottom surface **25** below the first surface **7** of the base wall. The side walls **27** of the well can also be tapered as shown in Figure 5. A well can also be formed by a cylindrical wall **29** projecting away from the first surface **7** for a distance less than that of the first section **15** of the outer wall as shown in Figure 6. The wells shown in Figures 4-6 are suitable for receiving smaller amounts of the biological material or where a deeper volume of material over a smaller area is desired in comparison to the entire dish surface area.

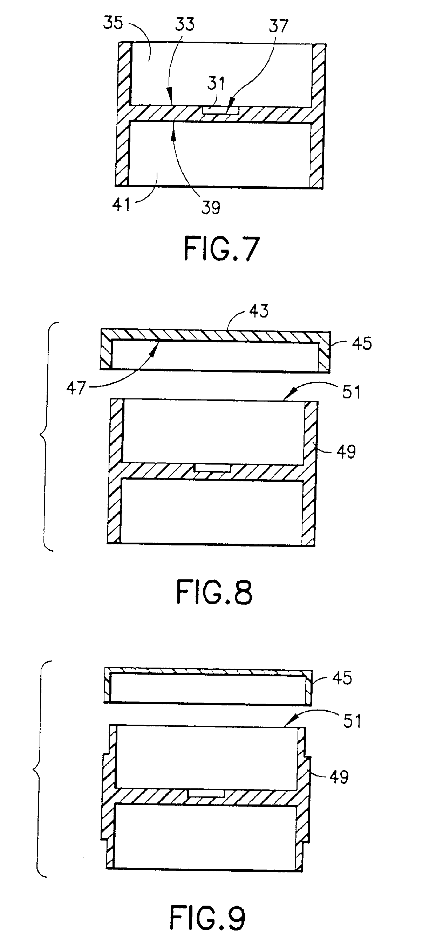

The reversible petri dish can include a circular base wall and a cylindrical outer wall which is integral with and encompasses the periphery of the circular base wall. The two oppositely facing dishes can be symmetrical, i.e., the same dimensions and volume with the exception of one dish containing a well. For example, referring now to Figure 7, there is seen a side elevation of a reversible petri dish having two symmetrical dishes, except for a cylindrical well **31** embedded into the first surface **33** within the first dish interior **35**. In such an embodiment the working surface of the first dish is generally limited to the bottom surface **37** of the well, while the working surface of the second dish encompasses the entire surface area of the second surface **39** within the second dish interior **41**. Other configurations that are contemplated, but not shown in the figures, include multiple wells in one dish, different numbers of wells in each dish and/or different size wells in each dish.

It should be appreciated that a particular dish can contain multiple wells and that the well(s) can be shapes other than circular or cylindrical, such as square, rectangular, oval, square or rectangular with rounded comers, concave indentations in the bottom surface of the dish, etc.

The reversible petri dish can also include a cover or covers that fit over one or both dish openings. With reference to a cylindrical shaped dish, the cover can be in the form of a truncated cylinder that fits over the opening of a dish. As seen in Figure 8, in a cross-sectional view, the cover includes a circular top wall **43** and cylindrical side wall **45** or skirt encompassing the periphery of the top wall **43** and projecting outwardly and substantially at a right angle from the inside surface **47** of the top wall. The inside diameter of the cylindrical side wall **45** of the cover is slightly larger than the outside diameter of the cylindrical outer wall **49** of the dish so that it fits over and covers the dish opening **51**.

In a preferred embodiment the wall thickness and outer diameter of the cylindrical outer wall **49** of the dish is reduced adjacent to the dish opening **51** to accommodate the cover as shown in Figure 9. The inside diameter of the side wall **45** of the cover is large enough to fit over the reduced diameter portion of the cylindrical outer wall **49.** The outside diameter of the side wall **45** is preferably flush with or only slightly larger than the outside diameter of the outer wall **49**.

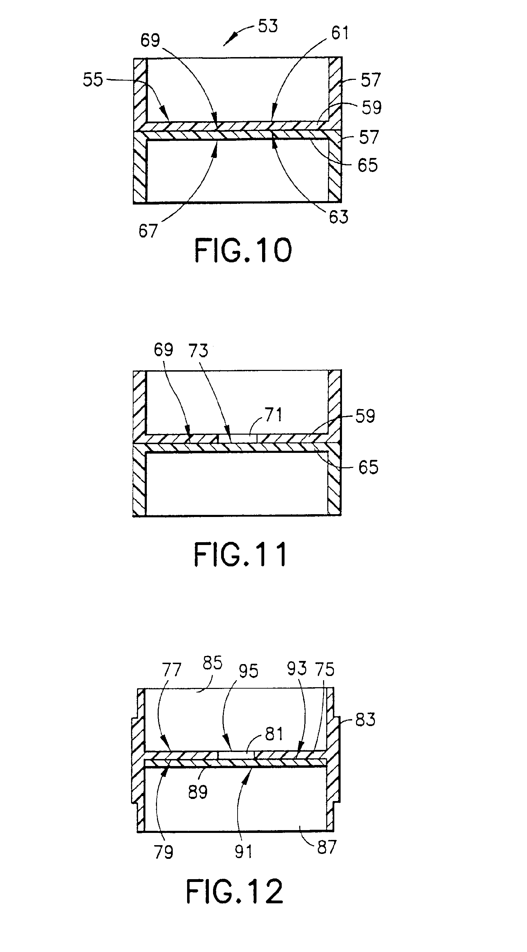

Referring now to Figures 10-11, side elevation cross-section views of preferred embodiments of the invention are shown. Figure 10 shows a reversible petri dish **53** which includes a circular surface supporting wall **55** and a cylindrical outer wall **57**, which encompasses the periphery of the circular supporting wall. The circular surface supporting wall **55** includes a composite of two layers. The composite includes a first surface layer **59** having a face side **61** and a back side **63**, and a second surface layer **65** having a face side **67** and a back side **69,** in which the back side **63** of the first layer **59** is adhesively attached to the back side **69** of the second layer **65,** wherein the face side **61** of the first layer **59** defines the bottom surface of the first dish and the face side **67** of the second layer **65** defines the bottom surface of the second dish. The first and second surface layers can be comprised of different materials, can have different surface structures or can have different surface coatings, depending upon the desired characteristics. A well **71** can be formed by a hole passing through the first surface layer **59** so that the closed end of the well **73** is the back side **69** of the second surface layer **65** as shown in FIG. 11. The first surface layer **59** is preferably comprised of a rigid polymeric material and the second working surface layer **65** is preferably comprised of glass.

Those skilled in the art will appreciate that many variations of the above-described embodiment of the invention may be made without departing from the spirit and scope of the invention. For example, referring now to Figure 12, there is seen a side elevation cross-sectional view of a preferred embodiment reversible petri dish of the invention which includes a circular base wall **75** having a first surface **77** on one side, a second surface **79** on the opposite side and a hole **81** passing through the central portion of the base wall **75**. The reversible petri dish also includes a cylindrical outer wall **83** integrally formed with the circular base wall **75** and encompassing the periphery of the circular base wall. The cylindrical outer wall **83** projects in a first direction outwardly and substantially at a right angle from the first surface **77** of the circular base wall to form a first dish interior **85** and projects in a second direction outwardly and substantially at a right angle from the second surface **79** of the circular base wall **75** to form a second dish interior **87.** The cylindrical outer wall **83** preferably includes a section of reduced wall thickness and outside diameter to accommodate a cover as described above with reference to Figure 9. The reversible petri dish also includes a circular transparent sheet **89** (or coverslip) having a face surface **91** on one side of the sheet and a back surface **93** on the opposite side of the sheet. The back surface **93** of the transparent sheet is adhered to the second surface **79** of the base wall, such that the sheet covers the hole **81** which passes through the base wall **75**, and extends to the periphery of the base wall **75** adjacent to the cylindrical outer wall **83.** The hole **81** passing through the base wall **75** and the back surface **93** of the circular transparent sheet **89** together define a well with an opening **95** facing into the interior **85** of the first dish, with the back surface **93** of the circular transparent sheet being the bottom surface of the well.

Preferably, the circular base wall **75** and the cylindrical outer wall **83** is formed as a one piece construction from a rigid polymeric material. The circular transparent sheet **89** is preferably comprised of glass, with the back surface of the sheet adhered to the second side of the base wall using an adhesive, e.g., a UV cured adhesive. In such an embodiment, a transparent window is formed over the bottom surface of the well.

In yet another embodiment, one or both working surfaces of the dishes, i.e., surfaces for receiving, growing or examining biological entities, can be coated with so called "biocoats" or "biocoatings" to support and/or control cell cultures. Biocoats can include any biocoat materials known in the art, such as collagen, amorphous collagen, native fibrillar collagen and native fibrillar collagen with butyric acid induction. For example, the face surface and/or back surface of the transparent sheet can include a biocoating.

Although the reversible petri dish has been described above with reference to embodiments having a circular shape, i.e., circular base wall and cylindrical side walls, it should be appreciated by those skilled in the art that the invention applies to other shapes such as oval, square, rectangular or other shapes used for petri dishes.

The reversible petri dish according to the invention is intended to be used for culturing micro-organisms or for visual assay of biological entities. The petri dish can be flipped to select a characteristic or structure for a particular use. For example, one side can provide a dish in which the entire surface area of the bottom of the dish is used as a working surface and the reverse side can provide a dish containing a smaller well in which the bottom surface of the well is used as a smaller working surface. In another example, the bottom surfaces of the two oppositely facing dishes can be the same (or similar) size, but have different physical characteristics, such as different materials, different textures or different coatings.

Those skilled in the art will appreciate the simple, unitary construction of the reversible petri dish and the different structural features. The improved efficiency for laboratory work requiring the use of petri dishes having different working areas or volumes is evident. The reversible petri dish can simply be flipped over to employ the desired dish structure. The design also prevents scratching or soiling of the back side of the working surface, preserving the optical quality of the dish for viewing biological samples using an optical microscope.

## Claims

1. A reversible petri dish comprising:
a base wall having a first surface on one side and second surface on the opposite side; and
an outer rim, said outer rim projecting in a first direction outwardly and substantially at a right angle from said first surface of said base wall to form a first dish interior and said outer rim projecting in a second direction outwardly and substantially at a right angle from said second surface of said base wall to form a second dish interior which is diametrically opposed to said first dish interior;
wherein the first and second dish interiors have different physical characteristics.

2. A reversible petri dish according to Claim 1, wherein different physical characteristics refers to different materials, different surface textures, different surface coatings, different surface area and/or volume for receiving biological material, or combinations thereof.

3. A reversible petri dish according to Claim 2, wherein different physical characteristics refers to different surface area and/or volume for receiving biological material.

4. A reversible petri dish according to Claim 3, wherein at least a portion of said base wall is comprised of a rigid, transparent material.

5. A reversible petri dish according to Claim 4, wherein said rigid, transparent material is a polymeric material or glass.

6. A reversible petri dish according to Claim 1, wherein at least one surface of said base wall supports at least one circular well having a well volume less than the respective dish volume.

7. A reversible petri dish according to Claim 6, wherein said circular well is imbedded into the surface of said base wall so that said well has a bottom well surface below the surface of said base wall.

8. A reversible petri dish according to Claim 7, wherein said bottom well surface is transparent.

9. A reversible petri dish according to Claim 8, wherein said bottom well surface is comprised of glass.

10. A reversible petri dish comprising:
a surface supporting wall having a first surface on one side and a second surface on the opposite side; and
an outer wall, said outer wall projecting in a first direction outwardly and substantially at a right angle from said first surface to a first open end to define a first dish interior, projecting in a second direction outwardly and substantially at a right angle from said second surface to a second open end to define a second dish interior, and encompassing said dish periphery;
said first surface comprising at least one well, said well having an open end, a closed end and a peripheral wall therebetween defining a well interior for accommodating a biological specimen and having a periphery within said dish periphery.

11. A reversible petri dish according to Claim 10, wherein the closed end of said well defines a first working surface area and the second surface defines a second working surface area.

12. A reversible petri dish according to Claim 10, wherein said surface supporting wall is comprised of a rigid, transparent material.

13. A reversible petri dish according to claim 12, wherein said rigid, transparent material is selected from the group consisting of glass, a polymeric material and a combination thereof.

14. A reversible petri dish according to Claim 10, wherein said well is embedded into said first surface so that the open end of said well is located at the first surface and the closed end of said well is below the first working surface.

15. A reversible petri dish according to Claim 10, wherein said surface supporting wall comprises a composite of at least two layers.

16. A reversible petri dish according to Claim 15, wherein said composite comprises a first surface layer having a face side and a back side, and a second surface layer having a face side and a back side, wherein the back side of said first layer is adjacent to the back side of said second layer, so that the face side of said first layer is said first surface and the face side of said second layer is said second surface.

17. A reversible petri dish according to Claim 16, wherein said well is formed by a hole passing through said through said first surface layer so that the closed end of said well is the back side of said second surface layer.

18. A reversible petri dish according to claim 17, wherein said first surface layer is comprised of a rigid polymeric material and said second surface layer is comprised of glass.

19. A reversible petri dish comprising:
a circular base wall having a first surface on one side and a second surface on the opposite side, and having at least one hole passing through the central portion of the base wall;
an outer rim integrally secured to said circular base wall, and encompassing the periphery of said circular base wall, said outer rim extending in a first direction outwardly and substantially at a right angle from said first surface of said circular base wall to form a first dish interior and projecting in a second direction outwardly and substantially at a right angle from said second surface of said base wall to form a second dish interior which is diametrically opposed to said first interior; and
a circular transparent sheet having a face side and a back side, said back side being adhered to said second surface of said base wall and substantially covering said second surface.

20. A reversible petri dish according to Claim 19, wherein said circular transparent sheet is comprised of glass.

21. A reversible petri dish according to Claim 19, wherein said back side of said circular sheet is adhered to said second side of said base wall using an adhesive.

22. A reversible petri dish according to Claim 19, wherein said hole passing through said base wall and said back side of said transparent sheet together define a well so that said back side defines the bottom surface of said well and said through hole defines the side walls and open end of said well.
